(19)

Europäisches Patentamt

European Patent Office

Office européen des brevets

(11) **EP 4 454 549 A1**

(12) **EUROPEAN PATENT APPLICATION**

(43) Date of publication:
**30.10.2024 Bulletin 2024/44**

(21) Application number: **23169779.8**

(22) Date of filing: **25.04.2023**

(51) International Patent Classification (IPC):
**A61B 5/024** *(2006.01)*  **A61B 5/00** *(2006.01)*
**A61B 5/055** *(2006.01)*

(52) Cooperative Patent Classification (CPC):
**A61B 5/02416; A61B 5/055; A61B 5/7289;**
**A61B 5/7292;** A61B 5/0073; A61B 5/7257

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **DEN BRINKER, Albertus Cornelis**
  **Eindhoven (NL)**
• **DER SARKISSIAN, Henri Henroutune**
  **Eindhoven (NL)**
• **WUELBERN, Jan Hendrik**
  **5656AG Eindhoven (NL)**

(74) Representative: **Philips Intellectual Property &**
**Standards**
**High Tech Campus 52**
**5656 AG Eindhoven (NL)**

(54) **SYSTEM AND METHOD FOR REMOTE PPG MR TRIGGERING**

(57)    A gating system (GT) and related method for data acquisition by an imaging apparatus (IA) in respect of a target biophysical system (TS). The system comprises an input interface (IF) through which is receivable a time-domain phase representation of a time domain signal of measurements of a surrogate biophysical system (SGS), obtainable by a sensor (S) device capable of non-ionizing operation. A biomarker extractor (BX) extracts from the time-domain phase representation an instance of a biomarker that is associable to a state of the target biophysical system. An output interface (OUT) provides, for a data gating operation, an indication for the instance of the biomarker being so extracted.

**FIG. 4**

EP 4 454 549 A1

**Description**

FIELD OF THE INVENTION

The invention relates to a gating system for data acquisition by an imaging apparatus in respect of a target biophysical system, to a related method, to an imaging arrangement including such as system, to a computer program element, and to a computer readable medium.

BACKGROUND OF THE INVENTION

Cardiac magnetic resonance imaging (MRI) typically uses triggering, based on electrocardiogram (ECG) measurements, for scans which require synchronization to the cardiac pulse and/or heart phase. Such synchronized triggering may also be referred to as some form of gating.

The ECG measurements may include the so-called R-peak, a signal feature, that represents electrical activity at the start of contraction of the heart muscle, ie, the R-peak indicates the start of the systolic phase. Based on the R-peak, an MR triggering sequence may be started. The triggering sequence may include one or more acquisition pulses and, optionally, a preparation pulse(s) (pre-pulse(s)). The total duration of this sequence is checked with duration of the heart cycle, and with intended image formation. If the sequence does not fit into the cycle, it may be split over two cycles making sure that the acquisition pulses occur at the desired phases of the cardiac cycle.

For various reasons, it may be desirable to exchange the ECG based measurements and triggering for other measuring modalities. For example, contactless camera-based Photoplethysmography ("PPG") triggering may be used instead of ECG , in order to improve workflow, to reduce likelihood for cross-contamination, to reduce waste (disposables), and to operate in higher MR-field strengths, etc.

Requirements for high-quality trigger derivation from such a PPG signal are high. Therefore, efforts have been spent lately to improve the PPG signal quality and the collection of derived markers associable with a MRI trigger event of interest, such as the said R-peak or other.

SUMMARY OF THE INVENTION

There may be a need for improved operation of an imaging apparatus, in particular for medical imaging.

An object of the present invention is achieved by the subject matter of the independent claims. Further embodiments are incorporated in the dependent claims. It should be noted that the following described aspect of the invention equally applies to the related method, to the imaging arrangement, to the computer program element, and to the computer readable medium.

According to a first aspect of the invention there is provided a gating system for data acquisition by an imaging apparatus in respect of a target biophysical system, the system comprising:

an input interface through which is receivable a time-domain phase representation of a time domain signal of measurements of a surrogate biophysical system, obtainable by a sensor device capable of non-ionizing operation;
a biomarker extractor capable in at least one extraction event to extract, from the time-domain phase representation, at least one instance of a biomarker that is associable to a state of the target biophysical system; and
an output interface for providing, for a data gating operation, an indication for the at least one extraction event.

Thus, when such biomarker is extracted (that is, such an instance is found or identified in the time domain phase signal using feature(s) of the signal), the timing of the extraction constitutes such an extraction event. And the fact that such extraction happened, is provided in the indication as output to so control drive or otherwise influence the gating operation. The providing of such indication happens preferably promptly, in real-time, that is (quasi-)concurrently when the event happens. Two types of such gating operations are envisaged herein: thus, in embodiments, the said data gating operation may be prospective or retrospective, wherein the prospective gating operation include triggering a data acquisition operation by the imaging apparatus, based on the provided indication of the at least one extraction event, wherein the retrospective gating operation includes tagging data acquired by the imaging apparatus, based on the provided indication of the at least one extraction event.

In embodiments, the sensor device is arrangeable for contactless operation. This allows for improved medical workflow.

In embodiments, the time-domain phase representation of the phase is computable by a transformer capable of effecting a Hilbert transform of the time domain signal of measurements. In some embodiments, the computing of the time-domain phase representation of the phase by transformer corresponds to such a Hilbert transform. Fourier methods may be used. No explicit computation of the Hilbert transform as such may be needed in some embodiments.

The phase may as per phasor in the complex plane, relative a reference, such as the real or imaginary axis. In embodiments, the time-domain phase representation is one of an instantaneous phase.

In embodiments, the time domain signal of measurements, on which the transformer is capable of acting on, includes plural frequency components. The signal may be assumed herein to be at least quasi-periodic signal. Thus filtering out essentially all frequency components but the fundamental is not preferred herein, as this appears to degrade system performance it has been found.

In embodiments, a timing of the extracted biomarker corresponds to a timing before a maximum or minimum occurs in the time domain signal of measurements. This has been found to be useful for cardiac imaging in particular.

In embodiments, the system comprises a filter capable of low-pass filtering the time domain signal of measurements, and/or of low-pass filtering the receivable time-domain phase representation. Noise filtering may be used in either instance, or in both.

In embodiments, the extractor is capable of such extraction operation based on a thresholding, such as zero-crossing of the time domain phase signal.

In embodiments, the extractor is capable of such extraction operation based on processing data (such as averaging, filtering, or otherwise combining data), of the time domain phase representation over a time-domain window.

In embodiments, an endpoint of such window is situated in time domain at a margin away from a current time of the time domain phase representation. This allows more robust processing, as intervals of signal degradation immediately trailing the current time can be avoided.

In embodiments, system may include, or may be capable of, interfacing with a control interface, the control capable to so cause triggering of an acquisition operation by the imaging apparatus, based on the provided indication of the at least one extraction event.

In embodiments, the imaging apparatus is of the tomographic type, in particular of the magnetic resonance type, or of the projection-domain type. However, other modalities, such as CT (computed tomography) )or nuclear are also envisaged, either in the alternative, or in combination such as in certain tandem systems for example.

In embodiments, the target biophysical system is capable of transitioning through states, the said state being one of such states.

In embodiments, the target or surrogate biophysical system includes any one or more of: i) the cardiac system, ii) the respiratory system, iii) and the gastrointestinal system. The cardiac system may be of interest in cardiac imaging gated for states of the heart's electrification, such states representable by feature of the QRS complex. In particular, the state may correspond to an R-peak (systolic state) or a diastolic state, etc. Of note however, no ECG is needed in the proposed setup.

In embodiments, the surrogate biophysical system includes a patch of skin of an imageable patient.

Specifically, in embodiments, the patch of skin is or at one or more of i) a human digit, ii) a head of an imageable patient. In PPG for example, patch of the forehead, such as at one of the temples may be used. In particular in systems with contact, a finger-tip clip type PPG sensor may be used.

In embodiments, the extracted biomarker corresponds to an instant in time domain of a presence of low (such as a minimum over the heart cycle) of blood volume at the surrogate biophysical system, such as at the said patch of skin.

In embodiments, the sensor device is configured for sensing radiation, such as any one of visible light, IR, NIR, laser (eg, LIDAR). The sensor may be PPG sensor or other, capable of acquiring of video that measures changing skin reflections caused by the changing blood volume there. However, non-radiation based sensor are also envisaged herein, such as ultrasound sensor, or sensor capable of sensing any one of: an acoustical signal, mechanical pressure or force. The latter may be used in particular when measuring the signal in respect of the respiratory system, or the gastrointestinal system. However, ballistocardiographic sensor or similar, may also be used to measure external manifestations of internal organs, such as recoil action caused by cardiac activity, etc.

In embodiments, the senor s arranged for remote operation, remote from any one or more of the target biophysical system, and/or of the surrogate biophysical system.

In another aspect, there is provided an imaging arrangement, including the system of any one of the previous claims, and further including one or more of i) the imaging apparatus, and ii) the sensor device.

In embodiments, the system includes a delay estimator module configured to estimate a delay between the timing of the extracted biomarker, and a timing of the state. In embodiments, the delay may be used to adapt instructions for the data acquisition that is to be gated for, based on the extraction event. For example, such instructions may include an MRI pulse sequence specification. The MRI pulse sequence specification may specify a trigger delay. This trigger delay may be adapted based on the estimated delay so that it can be better used for grating based on the extracted biomarker, extracted from the time domain phase representation.

Thus, in embodiments, the system may include a data interface to retrieve existing instructions for the data acquisition from a memory, and adapt same based on the estimated delay. The so adapted instructions for the data acquisition may then be used (instead of the existing one) for data acquisition in the gating operation. In particular, and in MRI, the MRI pulse sequence specification with trigger delay adapted based on the estimated delay may be used in the gating.

In another aspect, there is provided a (computer-implemented) gating method for data acquisition by an imaging apparatus in respect of a target biophysical system, the method comprising: receiving a time-domain phase representation of a time domain signal of measurements of a surrogate biophysical system, obtainable by a sensor device capable of non-ionizing operation;

> in at least one extraction event, extracting, from the time-domain phase representation, at least one instance of a biomarker that is associable to a state of the target biophysical system; and
> providing, for a data gating operation, an indication for the at least one extraction event.

In another aspect, there is provided a computer program element, which, when being executed by at least one processing unit, is adapted to cause the processing unit to perform the method.

In another aspect, there is provided at least one computer readable medium having stored thereon the program element.

In another aspect there is provided the use of the time-domain phase representation of a signal (such as PPG) for gating data acquisition operation in the imaging apparatus. The (underlying) signal is preferably other than an ECG signal.

Preferably, the said use includes using the estimated delay in the gating operation, in particular in the data acquisition operation.

What is proposed herein is to use a transformed version of, for example, a PPG signal (waveform), and to extract biomarker(s) ("marker(s)"), based on the time domain phase. The PPG signal is one example of the original signal of measurements. The instantaneous phase based on the analytical signal is one example of the said transformed version of the signal of measurements. Such transformed version may be based on the analytic signal representation for example. That is, as per the proposed system, instead of extracting a peak or other feature from the time-domain measurement signal (eg, the said PPG signal), a second signal (the said transformed version) is derived, which is a time domain phase, such as the instantaneous phase, as may be gotten as the phase of the analytical signal representation of the original signal of measurements. It has been found that this transformed version of the signal shows sufficiently stable periodicity and fidelity with respect to the heart pulse, and hence can be used as a surrogate for the PPG waveform in triggering or in other gating applications.

In the instantaneous phase signal, or other such time domain phase signal (which represents the (non-constant) phase as a function over time), a zero-crossing (or other definition/feature) have been found to constitute a stable marker(s) ) with respect to the R-peak, for example. The zero-crossing(s) have been found to occur a short time before a blood volume minimum state/R-peak, and are independent of the heart rate. This invariant property is desirable for MRI triggering, or for gating more generally. Instead of zero-crossing, any other thresholding may be used.

An ECG-blind synchronization with the R-peak feature is proposed herein in some embodiments. A low-order model may be used, based on PPG-signal features as represented by the marker in the time domain phase signal. In other words, with the proposed system, no ECG signal is needed and thus no ECG measurement equipment is required. An expected, patient-specific delay between R-peak and the marker may thus be determined at the start of the scan procedure. Thus, with the proposed system, gating is enabled (such as in MRI) based on the marker, with

> marker extracted from the time domain phase of the original measurement signal; marker occurs (timewise) earlier than a feature of interest, such as the maximum in the original signal of measurements;
> marker is responsive to details associated with systolic/diastolic phase; and marker is functionally coupled to R-peak feature, and may thus be used for "blind" prediction (without ECG)).

The above mainly relates to an application in cardiac imaging, but such marker may also be used for other cardiac imaging applications, or indeed for other than cardiac imaging.

The proposed method is particularly suitable for real-time processing so may be used for prospective gating, that is, for triggering of the data acquisition operation in/by the imaging apparatus. However, retrospective gating is not excluding herein.

The proposed method or system is suitable for real-time processing because of at least the following reasons:

> a physiological delay between occurrence of R-peak and marker is low;
> a algorithmic delay (latency) incurred for marker extraction is low; and
> the proposed setup is repeatable and robust, with jitter in marker extraction being low.

The above-mentioned delays (physiological or algorithmic) and the said jitter are low in the sense that they consume less than consumed by the changes that occur at target and/or surrogate biophysical system, and the time (latency) needed for the imaging (data acquisition operation) to conclude. Specifically, the method an system is well suited for gating (eg, for triggering) in MRI, as a whole sequence including pre-pulse, preparation time, and imaging pulse can be fit into an IBI (inter-beat intervals). Thus, the delay between for example an R-peak state, and trigger can be kept short, thanks for the proposed setup which uses the time domain phase for finding trigger point (biomarker).

The proposed system is mainly described with reference to the medical field, but this is not necessarily required herein in all embodiments, as the principles of surrogate observation by a sensor device, in lieu of measurements (directly) at the target system can also be applied in non-medical settings where the target system is not accessible for some reason. Having said that, whether in the medical field or not, whilst the target system may well be different, and remote from, the surrogate system that is used for the measurement to facilitate gating, this is not a necessity herein. Thus, in some application scenarios, the target biophysical system and the surrogate biophysical system may be one and the same, or one may at least include the other.

*"user"* relates to a person, such as medical personnel or other, operating the imaging apparatus or overseeing the imaging procedure. In other words, the user is in general not the patient.

*"time domain phase"*, this refers to an aspect of the time domain signal of measurements. The signal of measurements may be referred to herein for brevity as the *"measurement signal"*. The time domain phase captures an overall global phase for the signal at a given time instant, or up to the time instant. Thus, this phase is not tied to a particular frequency of the *measurement signal*. The time domain phase may relate to an evolution of a phase of an angular description of a phasor in the complex plane that can be associated with the measurement signal. Just like the measurement signal, it is also the phase representation that is in time domain, so can be used for gating purposes. It has been found that spreading out the information as encoded in the original measurement signal into the complex plane allows laying bare in greater conspicuity suitable bio-markers, especially for cardiac imaging. States of the surrogate system (such as per an over-time/time-dependent PPG signal, or other surrogate measurement signal) correlated to states (eg, cardiac states) of the target system have been found to yield suitable bio-markers, when the measurement signal is transformed into a representations in the time domain phase, and such transformed signal facilitates biomarker extraction for gating. In example, the original measurement signal is thus encapsulated into a complex signal (eg, the analytical version of the initial time domain signal, based on the Hilbert transform), or into a version of the measurement signal in higher than two dimensions, and its phase over time is considered herein instead of the original signal for gating purpose. As disclosed herein, the phase (angular argument) of the Hilbert transform based analytical version of the original measurement signal (such as a time series of optical or non-optical imagery) may be considered herein for gating in some examples.

*"surrogate (biophysical system)"* vs *"target (biophysical system)"*, the surrogate biophysical system and the target biophysical system may represent different parts (organs, anatomies, or groups or parts thereof), or functions (metabolic or physiological), or any other aspect of, or in, the human body that are related to each other. Thus, measurements taken from the surrogate biophysical system may be used to predict a state of interest of the target biophysical system, whilst the target biophysical system may not itself be accessible, or direct gating is not practicable, or cumbersome. In cardiac applications, states of the heart as the target may include R-peak or other features of the QRS complex, whilst states of the surrogate may include amount of blood at a part of skin, such as at forehead or finger of patient, etc.

*"biomarker"* (in short, *"marker"*), may include any property or feature of signal s or in its transform T(s) or that is manifest in the transform Ts), and the said property or feature is correlatable to a state (state of interest) and state change(s) in respect of the target system. The biomarker may change in appearance and quality as the surrogate system is co-changing with the target system. The state of interest may be a state one wishes to image for, but said state may also be of interest purely for gating, whilst one wishes to image another state or said imaging is at least not confined to the state of interest. In the latter case of being of state of interest for the sake of gating only, this approach may be useful to account for data acquisition latencies.

*"extraction"* of biomarker may include identifying a time instant when the biomarker is manifest and/or fulfills one or more conditions (extraction policy).

*"gating"* may be defined as synchronized processing of data acquired, or to be acquired, by imager of the target biophysical system. The target undergoes, or is subject to, change(s), thus transitioning between states. A state may be described as physiological configuration of the target system. The said processing is synchronized with the timing, that is, the occurrence of such states. Gating may be prospective or retrospective. Prospective gating as mainly envisaged herein amounts to triggering data acquisition at the right time based on the time instant of the extracted biomarker. Thus, frames are acquired only when the target biophysical system is in a predefined (same) state as per the time domain phase representation of the measurement signal acquired from the surrogate biophysical system. Retrospective gating amounts to acquire frames over time as if there was no motion or other state change that could impede image quality, but to tag each or some such frames with a respective tag representative of a state based on the time domain phase of the surrogate signal. This is done consistently over time so that frames that represent the biophysical target system in the same state receive the same tag. Then, once reconstruction is needed, frames for the desired state are retrieved based on the tags from the whole set of frames, and are reconstructed in one or more sectional image planes (volume) that represents the biophysical target system in the state of interest. No such tag-based retrieval is needed in prospective gating, as there, by definition, all frames are acquired only for the same state from the outset. Reconstruction can thus be done based on the acquired frames. Thus, in prospective gating, dose (such as in CT) and/or wear-and-tear on imaging apparatus components (such as forces on the coils in MRI) are reduced, as frames are acquired only when biophysical target system is in the right state (that is, in the state of interest for the imaging task at hand).

BRIEF DESCRIPTION OF THE DRAWINGS

Exemplary embodiments of the invention will now be described with reference to the following drawings, which, unless stated otherwise, are not to scale, wherein:

Fig. 1 shows a medical imaging arrangement for imaging a biophysical target system;
Fig. 2 shows an MRI imaging modality as may be used in the arrangement of Fig. 1;
Fig. 3 shows a block diagram of a system for controlling an MRI operation based on gating;
Fig. 4 shows a block diagram of a gating system as envisaged herein in embodiments and as may be used in the arrangement of Fig. 1;
Figs. 5,6 show time curves of measurements and transforms of such measurements to illustrate operation of the gating system of Fig. 4;
Fig. 7 illustrates a windowing operation which may be used in embodiments of the gating system of Fig. 4;
Fig. 8 illustrates signal features as may be used in the proposed gating system;
Fig. 9 shows a flow chart of a computer-implemented gating method as may be used in particular in medical imaging of a biophysical target system, and
Fig. 10 is an illustration of effects of the proposed method and gating system in the context of MRI.

DETAILED DESCRIPTION OF EMBODIMENTS

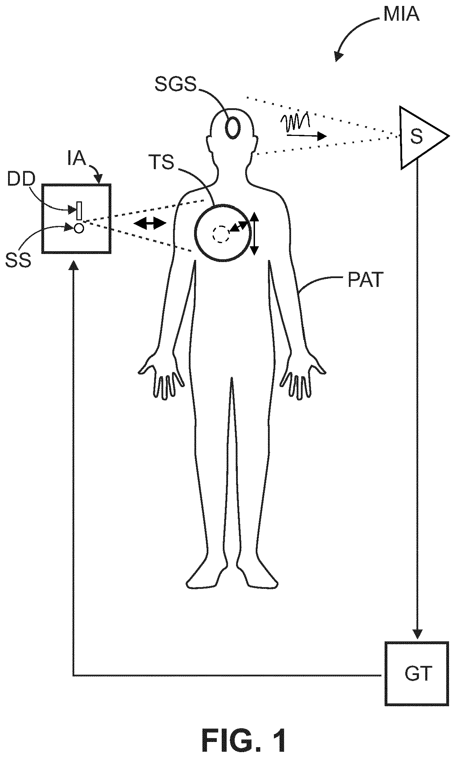

Reference is now made to the schematic block diagram of Fig. 1, which illustrates components of a medical imaging arrangement MIA. As envisaged herein, the medical imaging arrangement MIA uses a new type of gating administered by a gating component or gating system GT. In embodiments, the gating system GT is operable to synchronize operation of a medical imaging apparatus IA ("imager") with measurements collected by a sensor device S. The measurements form a measurement signal s and this is specifically transformed as envisaged herein. The gating system GT operates on the said so transformed measurement signal for better robustness and accurateness of the synchronization, as will be explained in more detail hereinbelow.

A medical objective supported by the gating component GT is to acquire good quality imagery $\varepsilon$ of a target biophysical system TS of a patient PAT. The target biophysical system TS may be an organ, anatomy, tissue, or a group of any of the aforementioned, on or within patient PAT. For example, the target biophysical system TS may include the heart of a human or animal patient PAT, such as in cardiac imaging. The below examples will indeed mostly be drawn from cardiac imaging, with the understanding that the principles described herein may also be used for other medical imaging tasks, not necessarily related to the heart. Even non-medical applications may not be excluded herein, as will become apparent below. Having said that, the gating principles disclosed herein have been found to work particularly well in a cardiac imaging setup as they seem to correspond well to some structural and/or dynamic properties of some such target biophysical system TS, such as the human heart. The gating component GT helps ensure image quality ("IQ") as the target biophysical system TS may undergo changes (such as motion), which, if not accounted for, may corrupt IQ. For example, such motion may cause image blur or other image artifacts. The gating system GT is configured to facilitate avoiding such IQ degradation.

At times, when one wishes to acquire imagery with an imaging apparatus IA of such a biophysical system TS, it may be necessary to monitor a surrogate biophysical system SGS, different, but functionally related to, the target system TS.

The imaging apparatus IA as envisaged herein is preferably configured for non-invasive imaging. The surrogate biophysical system SGS may be monitored by a suitably arranged sensor device S which, preferably remotely and non-invasively, acquires the said measurement signal as a time series $s=s(t)$. Thus, the said time series s of measurements (an over-time signal) is time domain data. The time domain measurement signal s so acquired may be used by the gating component GT to synchronize certain imaging tasks, such as triggering a data acquisition by the imaging apparatus IA to acquire data of the target system TS from which imagery of the target system TS is obtainable, such as by reconstruction or other. The above described use of the time domain measurement signal s for data acquisition synchronization by imager IA is an example of prospective gating, which is mainly envisaged herein. However, other types of gating, notably retrospective gating, is not excluded herein, and is equally envisaged. Such retrospective gating may include tagging, based on the measured signal s, a stream of data ("frames") acquired by imager IA. That being said, it is mainly prospective gating that is mainly envisaged herein.

As briefly mentioned above, a situation envisaged herein is that the biophysical target system TS is subject to changes as schematically indicated in Fig. 1 by the dotted circle and the double arrows. That is, biophysical target system TS is assumed to transition through plural states, such as in a periodic or at least quasi periodic manner, although non-periodic such manners are not excluded herein. The changes may happen during data acquisitions, or in between acquisitions and this may cause data inconsistencies and hence imagery artifacts, if it was not for the operation of the gating system GT. Motion of target physical system TS is an example of such a change, and is primarily envisaged herein. For example, in the context of motion, such state changes may be changes of position and/or of shapes of the target system TS as its transitions from state to state. For example, one state change may be in connection with deformation. Thus, the target system TS may expand or contract. In addition, or instead, the target system TS may be translated and /or it may rotate, or indeed may undergo changes that amount to a combination of any two or all of the aforementioned. The change(s) may happen repeatedly, or may be one-off. However, the proposed principles, whilst they mainly relate to motion type states and changes, such physical motion is not necessarily envisaged herein in all embodiments. Thus, in addition or instead of motion functional, metabolic or physiological changes are also envisaged herein. Such changes may be measurable by functional imaging such as SPECT/PET, MRI and other imaging modalities IA. Such imaging may be even down to a cellular, subcellular, or indeed molecular level (sometimes aided by the use of contrast material), and such are also envisaged herein in additional, or in alternative, embodiments.

As an example, and with continued reference to cardiac imaging, the heart TS undergoes during the cardiac cycle multiple states including, for example the systolic or the diastolic state. We shall refer to states in this connection rather than "phases", because we wish to reserve the term "phase" for a particular aspect of signal processing as will become apparent below. Broadly, the gating system GT allows accounting for transition between the states of the human heart, as otherwise image quality may be degraded by blurring or other artifacts that may even render the imagery $\varepsilon$ non-diagnostic in certain cases. Data acquisitions in prospective gating are triggered at predefined instants, when a certain state of interest in the target system TS is assumed to be manifest, given the measurement data collected at the surrogate system SGS. Otherwise, if the state/state changes are not accounted for, an imaging re-run may be then needed, which puts a burden on equipment, patient and staff, incurs additional costs etc, all of which can be avoided thanks to the gating system GT. In the prospective gating embodiment, the proposed gating system GT allows synchronizing the imaging operation, in particular triggering the data acquisition, based on the measurement signal s acquired by the sensor device S of the surrogate biophysical system SGS. Measurements at the surrogate biophysical system SGS may thus be associable with certain states of interest at the target TS. The target state may be of interest as such, as one wishes to image the ROI at that state, or the target state is of interest as this is when data acquisition/tagging is to start in order to image a certain aspect of the target, not necessarily confined to the state of interest. This allows accounting in particular for data acquisition latencies. Thus, in cardiac imaging, the R-peak may be as state of interest for imaging systolic state of the heart, but the R-peak may also be used as state for interest for gating when one wishes to image for other states of the heart, using, for example, certain pulse protocols in MRI that may cause some latency.

The sensor device S may be arranged as a PPG camera, and may be used to acquired video footage s of a certain patch of skin SGS of patient PAT. The patch of skin is subject to fluctuations (pulsation) in blood accumulation. Such pulsation cause color changes in the footage between frames, which can be ascertained by using a suitably sensitive light sensor in the PPG camera. The PPG signal may be understood to represents the oscillation (time-dependence) of reflection of the skin as a response to blood circulation /volume changes at the patch of skin. As illustrated in Fig. 1, such skin patch on the forehead of patient PAT has been found to yield good results. The pulsation is caused by the blood circulation which is driven by the heart. Specifically, the pulsation is an alternating sequence of states of blood accumulation and depletion caused by the pump action of the heart, in turn causing different optical coloration (spectra) in the video footage being recorded over time. Such PPG video footage has been found to capture well such state changes (coloration) of the patch of skin. Such patches of skin have thus been found to be a good surrogate biophysical system SGS for the cardiac system as the biophysical target system TS. States changes at the patch of skin SGS correspond in good approximation to the different states through which the heart TS is transitioning.

For example, the state of electrification of the of heart as may be recordable by an ECG trace has been found to be correlated with the blood volume state that transitions between blood depletion (low blood volume state) and accumulation (high blood volume state) at the patch of skin, and the various stages inbetween. The low blood volume state at the patch of skin SGS may be measured as troughs of a suitably coded PPG signal s(t). The beginning of the systolic state of heart, which is commonly identified by an R-peak in the ECG trace, has been found to correlate well with the low blood volume state in the PPG measurement signal.

In general, what is preferably used herein by gating system GS is a functional correlation, known or estimable, preferably stable, between the target system TS and the surrogate system SGS. In other words, as the target system TS is assumed to be transitioning through states, so is the surrogate system as this will also undergo associated state changes. However, the states of the surrogate system SGS may be of a completely different nature as states and its changes at the target system TS. For example, in cardiac imaging, the heart state may relate to a particular spatial configuration or electrification, of the myocardium. For example, the state may be a full or partial contraction, or may be a state of full or partial compression, etc, whilst the associated states at the patch of skin measured by the sensor S device relates to changes in color. The sensor S device may use a suitable CCD (charge-coupled device) sensor component for example.

Broadly, what is proposed herein is a hi-fidelity extraction by a component of the gating apparatus GT (to be described in more detail below) of a bio-marker b based on the time series of measurements s acquired by the sensor device S, and to use this for gating purposes such as for triggering image acquisition, or for tagging acquired data, respectively. More specifically, good results have been achieved herein by transforming the time domain measurement signals s first into a time domain phase representation of the signal, and to extract the bio-marker b from there.

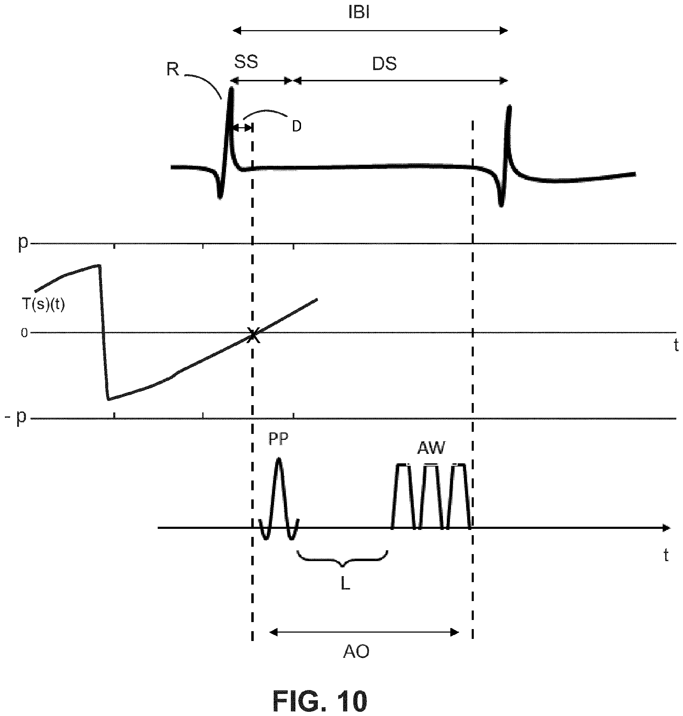

The bio-marker b itself may directly represent the desired state of the target system, but in some instances this may not necessarily be the case, so long as there is a known modellable, pre-defined, and sufficiently stable, relationship between the occurrence of the bio-marker and the state of interest of the target system for which one wishes to image. For example, it may well be that the occurrence $t^*$ of bio-marker b is either trailing or leading the occurrence of the state of interest of the target system. However, because this delay or advancement between the two can be modelled, or is known beforehand, and is preferably stable over time (low or no jitter), gating can be done reliably. Also, the state of interest may persist for time, so a sufficiently low delay still allows for gating in respect of the said state. Because the proposed processing setups are casual, real-time processing is facilitated. For example, as has been found herein in some embodiments for cardiac imaging, the state of interest is the heart's electrification state, in particular the occurrence of R-peak in an ECG, that is, the beginning of the systolic state. For example, the corresponding biomarker b, a low blood volume state as imaged by sensor device S at the patch of skin, and as represented in the time domain phase signal $T(s)$ into which s has been transformed into, has been found to generally occur after the instant of the R-peak. The representation of the low blood volume biomarker in the time domain phase presentation is surprisingly pronounced, which allows reliable extraction. In addition, the delay between the biomarker instant the R-peaks is sufficiently low, which is useful for MRI (see Fig. 10 below for more details on this). And what is more is that the said delay $\Delta$ has been found to be surprisingly stable in the time domain phase signal. The delay may be accounted for, if need be, by modelling a suitable time offset, which may be added to determine the gating signal (such as the data acquisition trigger for imager IA). Thus, imaging may be triggered at a time $t'= t^*+\Delta$, that is, at the time the bio-marker is detected, plus the time offset. The delay $\Delta$, such as between R-peak and the biomarker, can be modelled separately herein. If the biomarker b instant $t^*$ occurs after sate of interest, such as in the R-peak case for cardiac imaging with PGG, the instant $t^*$ can still be used for triggering, as this delay has been found to be sufficiently low for triggering the imaging acquisition.

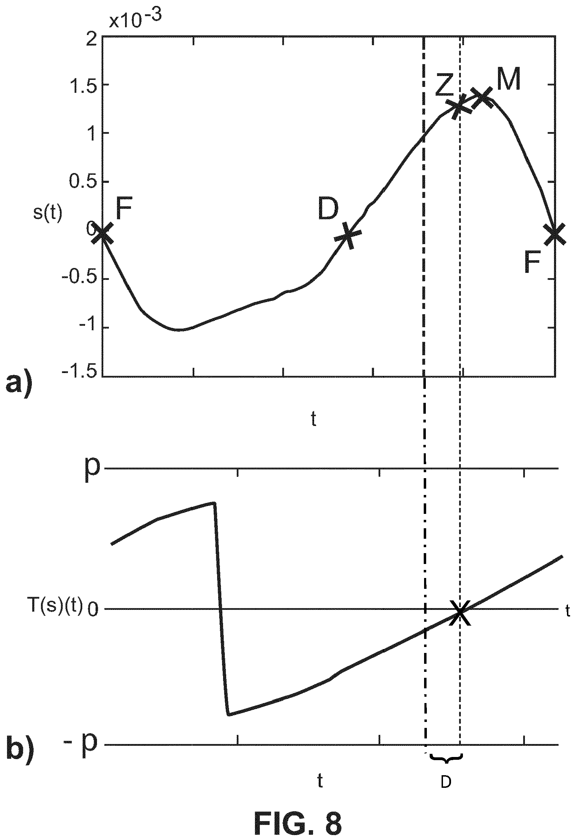

The delay may be assumed to be a constant across patients, or may itself be derived from prior measurements calibrated to a given patient individually. The delay may be learned from machine learning, or in any other way. It has been found that simple low order model, that is one using for example, one, two or three parameters, is sufficient to model this delay $\Delta$ (see below at Fig. 8 for more details).

Before describing operation of the gating system GT as envisaged herein in more detail, the imaging set-up MIA in terms of the imaging apparatus IA and the measurement sensor device S for surrogate system SGS is described first, in order to aid the subsequent explanations of gating system GT.

Turning now first to imaging arrangement MIA more generally, imager IA includes detector device DD and signal source SS. The imager IA is operable and configured to acquire using signal source SS, data $\lambda$ at its detector device DD of the target system TS. The data $\lambda$ so acquired may be used to obtain imagery $\varepsilon$ of target system TS. A stream of image data $\lambda$ ("frames") may be acquired, and a counterpart stream of such imagery $\varepsilon$ may be obtained from such data $\lambda$. The imager is preferably of the tomographic type and the obtaining of the imagery $\varepsilon$ from acquired data $\lambda$ (projection domain data) may include transforming from projection domain to image domain, such as by using a reconstruction algorithm implemented on a computing platform RECON. The surrogate sensor device S may include such a signal source in some embodiments, whilst in other embodiments ambient signals may be used. Thus, in some embodiments, the sensor S does not include such signal source SS, as it used ambient signals, such as the PPG or similar, whilst the imager IA does include such as signal source SS.

At any rate, the sensor device S is capable of transducing a property of interest of the surrogate system SGS into the time domain measurement signal s, buy using a sensor component. The surrogate sensor device S may itself be image-based, but this may not need be so in all embodiments. At any rate, the measurement principles, transducer mechanism and imagery (if any) acquired by the sensor device S is in general different from data acquired by the IA imager and the imagery $\varepsilon$. The sensor measurements s as acquired by sensor device S may be of a completely different nature in terms or clinical meaning/aspect, contrast etc. For example, in some embodiments the imager IA is configured for MRI imaging, whilst the sensor device S is configured for contact or, preferably contactless/remote, PPG imaging.

Turning now first in yet more detail to the surrogate biophysical system measurement sensor S device (also referred to herein simply as "(surrogate) sensor S (device)"), this may include a transducer sensor component (not shown) to acquire raw data $s'$, and, optionally, a pre-processor configured to pre-process the raw data $s'$ to obtain the measurement signal s to be then used by the gating system GT. The pre-processing may be done on-board the device S, or the preprocessing may be offloaded onto on external computing device, such as on the gating system GT itself, or on another computing device of the imaging arrangement MIA, or beyond.

As said, the sensor device S may be arranged as a PPG camera. However, other camera types, either configured for visible light or for other (non-visible) parts of the electro-magnetic-radiation spectrum are also envisaged herein. Indeed, other camera types may be used such as infrared (IR), near-infrared (NIR), or laser such as LIDAR, depth sensing, or any other.

In yet other embodiments, the sensor device includes a nuclear imaging apparatus or other imaging apparatus configured for functional, metabolic, molecular, etc, imaging. The sensor may be an imaging apparatus such as apparatus IA, but of different modality than imager IA. For example, if imager IA is MRI, sensor S device may be a CT apparatus, for example.

The surrogate sensor device S may not necessarily be image-based, or may not even be radiation based at all, depending on the nature of the surrogate signal s that one wishes to measure and that has the requisite correlation with the states that the target TS is assuming in its transitioning.

For example, in alternative embodiments of sensor S, this may be an acoustic one, such as an ultrasound sensor. In other, alternative, embodiments, sensor S may be configured for mechanical measurements instead, such as ballistic measurements. In example of this is ballistocardiography, where the pump action of the heart is associated with mechanical recoil impulses that can be measured. For example, the sensor device S may be arranged as a suitably sensitive scale device, an electro-mechanical sensor (eg, a piezoelectric element), etc, on which patient is placed. The sensor device S may be embedded in a chair or bed, stretcher, etc, in or on which patient sits or lies. The sensor device S responds to recoil action as are propagated through the body and caused by the cardiac pump action. Alternatively, depth sensing camera may be used to measure the recoil action by tracking movement (eg up and down when patient is in a supine position) of the abdominal or chest wall. Other sensor types envisaged herein include those configured for any one of phonocardiography, apexcardiography, seismocardiography, kinetocardiography, and others still. For an overview of such techniques, see the article by Laurent Giovangrandi et al in "Ballistocardiography - A Method Worth Revisiting", published in Conf. Proc. IEEE, Eng MedBiol. Soc, pp 4279-4282 (2011).

In addition, acoustic or RF sensor S may be used that responds to cardiac or respiratory states. An example is a Pilot Tone (PT) setup, which is based on the principle that patient motion (ie, due to breathing or cardiac activity) may cause coil-dependent variation of a signal amplitude as. A dedicated transceiver or transmitter/receiver setup can be used that sends/receives a dedicated RF signal into bore of imager IA, and thus for interaction with patient PAT. A dedicated hardware setup is not necessarily needed however in all cases, as in MRI, the coils there may be so used, controlled to send and/or receive, such a dedicated motion detector signal, different from the RF pulses used for MRI imaging. A similar setup may be used, but based on ultrasound or sound waves at other frequencies that respond to patient motion.

However, whilst such other sensor setups are indeed envisaged herein in some embodiments, it is mainly image-based arrangement such as the PPG (photo-plethysmogram) camera, or others that are of main interest herein, not lest for ease and cost-effective implementation. Accordingly, the below description will focus mainly on such embodiments, with the understanding that this is illustrative and not at the exclusion of the other mentioned embodiments of sensor S.

Instead of measuring blood volume or related quantities, such as skin reflection of light spectrum at a patch of skin in PPG, other surrogate system SGS using different sensor S may be used, when imaging for example for the respiratory system or the gastrointestinal system as target TS. Spirometer setup S may be used to measure lung states for lung imaging. Other such setups may include an elastic belt with associated pressure monitoring sensors, which may be fit over the chest. Measurement markers placed on patient's chest wall may be used instead which are observed by a camera. Imaging of the chest wall without specific markers placed are also envisaged, using as sensor S a depth sensing camera for example, with the benefit of being contactless. In imaging tasks in relation to the gastrointestinal system, peristaltic manometry may be used to allow gated-imaging of motility states of portions of the gastrointestinal system (esophagus, stomach, intestines), although this may be invasive.

Turning now in more detail to the imager IA, its signal source SS generates a signal, for example an interrogating signal, which interacts with the patient tissue, to produce a response signal which is then measured by the detector device DD as detector data $\lambda$, from which the said image domain medical imagery $\varepsilon$ is obtainable.

For example, in a CT setting, during an imaging session, the signal source includes an X-ray source SS that rotates around an examination region ER with the patient in it to acquire projection imagery from different directions. The projection imagery is detected by the detector device DD, in this case an X-ray sensitive detector. The detector device DD may rotate opposite the examination region with the X-ray source SS, although such co-rotation is not necessarily required, such as in CT scanners of 4th or higher generation. The signal source SS, such an X-ray source (X-ray tube), is activated so that an X-ray beam issues forth from a focal spot in the tube during rotation. The beam traverses the examination region and the patient tissue therein, and interacts with same to so cause modified radiation. The modified radiation is detected by the detector device DD as intensities. The detector DD device is coupled to acquisition circuitry, such as DAQ unit, to capture the projection imagery in digital domain as digital data $\lambda$. Radiographic imaging which operates in projection domain only, is not excluded herein.

In MRI embodiments, the signal source SS is formed by radio frequency coils which may also function as detector device(s) DD, configured to receive, in receive mode, radio frequency response signals emitted by the patient residing in a magnetic field. Such response signals are generated in response to previous RF (radio-frequency) signals transmitted by the coils in transmit mode. There may dedicated transmit and receive coils however in some embodiments instead of the same coils being used in the said different modes as transceiver devices.

As another example, in emission imaging, the source SS is within the patient in the form of a previously administered radio tracer which emits radioactive radiation that interacts with patient tissue. This interaction results in gamma signals that are detected by detection device DD, in this case gamma cameras, arranged preferably in an annulus around the examination region where the patient resides during imaging.

In preferred embodiments, cardiac imaging or imaging for other organs such as lung/chest imaging, abdominal imaging, etc, is done using for example an MRI imager IA or CT, or nuclear imaging. Ultrasound ("US") imaging modality IA is also envisaged herein in some embodiments.

The patient PAT may assume any position during operation of the imager and/or of the sensor S: Thus, patient may stand , lie, squat, etc, depending on the clinical practice and protocol and imaging modality used.

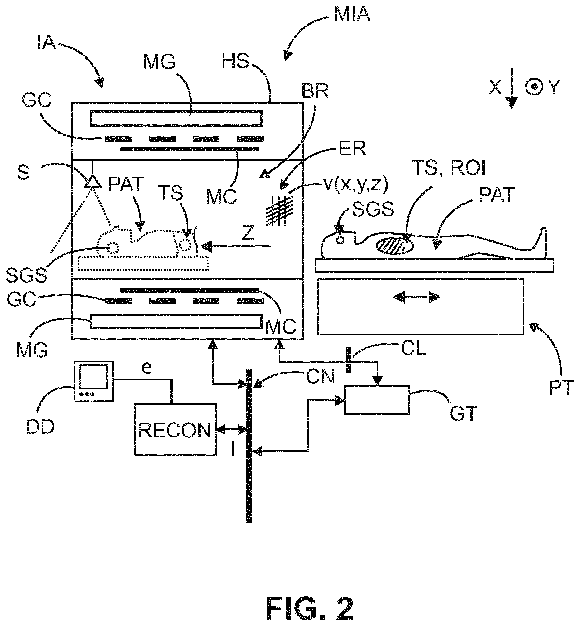

As an example, an MRI setup IA is schematically illustrated in Fig. 2, as one embodiment envisaged herein. In MRI (but also in CT), the patient PAT is slid, eg on a patient table PT, into a bore BR of imager IA defined by housing HS. The bore BR defines the examination region ER of imager IA in which patient PAT, or at least the region of interest RO (head, arm, heart, etc), resides during imaging. "Imaging" may also be referred to herein as "data acquisition", both terms being used herein interchangeably.

The examination region ER, and hence the 3D space therein occupied by patient PAT, may be thought of conceptually as made up of a grid of spatial points $(x,y,z)$. The imager IA acquires in the data acquisition/imaging operation, the said detector data $\lambda$, from which image values v are obtainable by reconstruction as implemented by reconstructor RECON. The reconstructor RECIN may include a computing device running a reconstruction algorithm. The algorithm may be applied to the acquired detector data $\lambda$. The acquired detector data $\lambda$ may also be called herein "projection data" $\lambda$, as opposed to the reconstructed image $\varepsilon$ which is in image domain. Thus, the reconstruction algorithm assigns image values $v$ to the voxels to obtain a set of spatially distributed voxel image values $v(x,y,z)$, thus building up a 2D, 3D or 4D imagery $\varepsilon$ of the region of the interest ROI, that is, of the target biophysical system TGS, such as of the heart or other organ (or groups of organs/anatomies) of interest for the given imaging task.

In prospective gating, data acquisition starts once a suitable control signal is received through a suitable control interface SL, based on trigger signal(s) established by the gating system GT. Most conveniently for cardiac imaging in MRI, the surrogate biophysical system SGS may be the mentioned patch of skin on the patient's forehead or on one of the temples, etc, as required. Such as surrogate system SGS is easily accessible for a line-of-sight-type based sensor device S. The sensor device S may be mounted inside or outside the bore BR, as illustrated in Fig. 2. The sensor may be set at an end portion of the bore BR, or beyond, such as being mounted on an external stand, on the wall or ceiling etc, of the examination room, with its field of view ("FOV") adjusted so it can capture the surrogate system SGS, such as patient's forehead whilst patient PAT resides in the bore during the imaging session.

Basic operation of the MRI imager rests on triggering, at suitable time instant, based on a timing signal provided by the gating system GT, a suitable pulse sequence by causing radio frequency signals issuing forth from one or more main coils GC. There may also be a mobile RF coil SC, arranged closer the ROI within the bore BR at patient's body.

Main magnetic field coils MG are disposed inside the housing HS. Main magnetic field coils may be generally of solenoidal configuration to produce a main $B_0$ magnetic field directed along a Z-direction lying parallel to a central axis of the scanner bore BR. The main magnetic field coils are typically superconducting coils disposed inside in cryo-shrouding, although resistive main magnets can also be used. Particularly at higher field strengths and therefore higher frequencies, superconducting magnets are preferred. Whilst Fig. 2 shows a closed cylindrical design, this is not a requirement herein as open design MRI scanners with U-shaped magnets are also envisaged herein, although the principles of what is proposed herein will be of particular benefit to closed design MRI scanners due to their restricted patient space as will become apparent below.

The housing HS may also house or support the gradient coils GC for selectively producing magnetic field gradients along the Z-direction, and/or along in-plane directions transverse to the Z-direction (such as along Cartesian X-and Y-directions), or along other selected directions.

The housing HS also houses or supports the radio frequency head or body coils MC (referred to herein as above as main coils) for selectively exciting and/or detecting magnetic resonances. Although birdcage coils are common at 128 MHz and below, other coils besides a birdcage coil can be used as a volume transmit coil, such as a transverse electromagnetic (TEM) coil, a phased coil array, or any other type of radio frequency coil. The housing HS typically includes a cosmetic inner liner defining the scanner bore BR. Broadly, the main magnet MG causes alignment of hydrogen nuclei (protons) in patient tissue and within its magnetic field $B_0$. The RF pulse emitted by RF coil MC disturbs this alignment. Once RF pulse is switched off, disturbed nuclei relax back into alignment, thus emitting during their relaxation, the resonance pulses which are picked up by coils MC in receive mode as the (in this case, RF) projection data $\lambda$. Contrast in such MRI imagery corresponds, that is varies with, spatial proton (Hydrogen nuclei) distribution in the examination domain ER, and hence on or within the patient's body/tissue. Imaging for other odd-numbers Z elements (that have an odd number of protons and neutrons) can also be done if needed. However, imaging for Hydrogen density (its protons) is very common, but this is for illustration only, as MRI based on response signals from other elements with unpaired nuclei, such as $^{13}C$, $^{14}N$, $^{19}F$ or $^{31}P$ are also envisaged herein.

The MRI pulse signals may be initiated based on a timing of an extracted target bio-marker b is established by gating system GT based on the measurements s acquired by camera S.

The pulse sequence may then be initiated, factoring in a possible preparation time, to acquire over a certain period suitable projection data $\lambda$ as response RF signals picked up by the RG coils as described above. The RF coils MC may thus be switchable between transmit mode and received mode. Alternatively, two set of coils RF are used, receiver and transmitters. A set of gradient coils GC may be used to localize the resonance response signal spatially in examination region ER. From the picked-up RF resonance projection data $\lambda$, sectional or volume imagery $\varepsilon$ in image domain of, eg, of the human heart in a desired state (eg, systolic) can be reconstructed by reconstructor RECON. This can be repeated over multiple heart cycles, until enough projection data $\lambda$ is acquired. Thus, the sectional image/image volume $\varepsilon$ may be obtained by reconstruction from the projection data $\lambda$ so acquired at the right instants, that is, synchronized with the state changes of the heart, using the measurements by sensor S of the surrogate system SGS as a telltale as described above in connection with Fig. 1. The MRI reconstruction algorithm may include an implementation of an inverse 2D Fourier transformation, or may use iterative reconstruction. In CT, filtered back-projection (FBP), or others, such as iterative approaches, algebraic, statistical, etc, may be used.

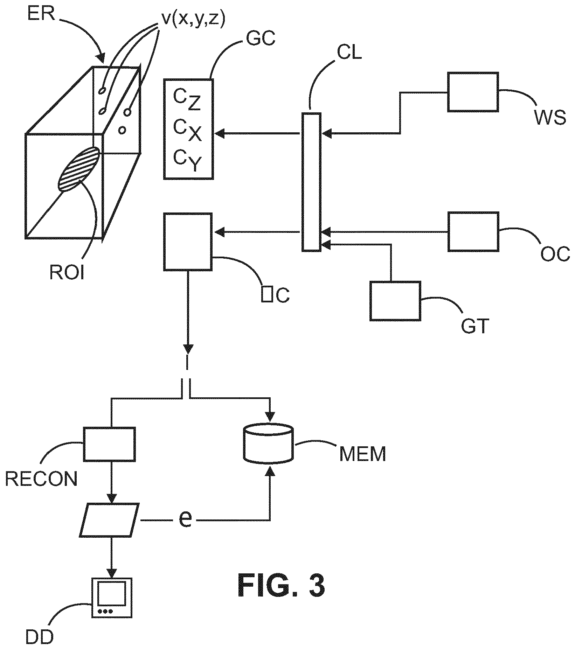

Reference is now made to Fig. 3 which briefly summarizes the imaging operation as synchronized by operation of the gating component GT in connection with MRI imaging. The RF coil MC is operated to issue, in transmit mode, radio frequency pulses into the examination region for interaction with protons in the human tissue resident in the image domain, with RF coil MC switched to receiver mode receive resonance response signals from the protons. The response signals are the data acquired during a scan operation of imager IA, from which the sectional image $\varepsilon$ of the spatial distribution of the Hydrogen protons can be reconstructed by the reconstructor RECON. The gradient coils GC have a different function. The gradient coils GC are operated for spatial localization of the resonance response signals caused by the RF coil MC. The gradient coils GC and RF coils are operated in concert according to scan timing protocol. As prescribed by the imaging protocol or imaging task at hand, at suitable timing, suable currents are applied to any one of the three sets CZ, CX, CY of the spatial gradient coils GC, and to the RF coil to cause excitation pulses. Switching on the main coils or surface coil is via control interface CL which is operated based on timing signals received from gating system GT. Gating system GT may be integrated into imager IA, for example into imager's IA operation console OC, especially for prospective gating. In retrospective gating, the gating system GT may be integrated into a work-station WS computer system, or into any other computing system.

A number or pulse protocols to be used by main coils MC (and/or a local coil placed at/on patient's body) to configure the excitation RF pulses. In some cases, in particular for cardiac MRI, such imaging sequence may include a pre-pulse, a waiting time called the preparation delay/latency, and the imaging window including the actual acquisition pulses. The preparation latency depends on the physical properties of the tissues one wishes to image for, and the length is usually a given constant which cannot be changed.

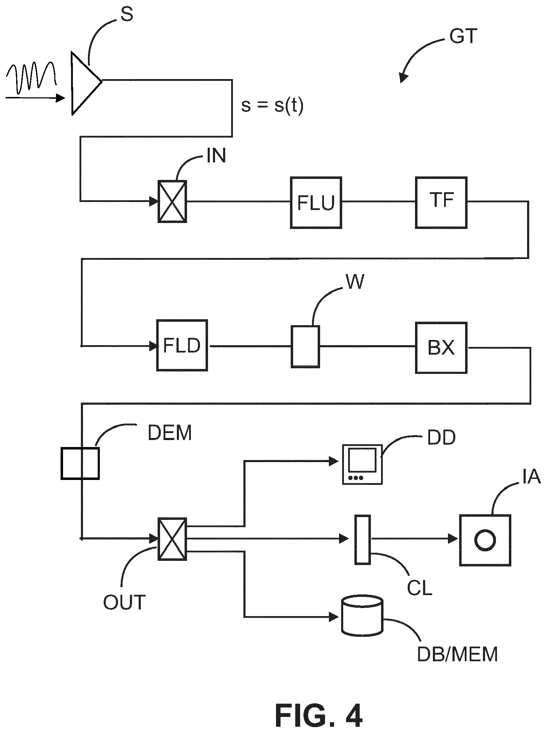

Reference is now made to Fig. 4, which shows a block diagram of the gating system GT. The gating system GT will now be described in more detail.

Surrogate sensor S device is operable to acquire the measurement signal taken from the surrogate biophysical system SGS as a time series $s=s(t)$. The measurements s may be of a state/quality of the surrogate system, and may include imagery/video, sound signal, scale measurements, etc. The signal is collected remotely and preferably contactless/non-invasively. In preferred embodiments, video feed of or in respect of surrogate biophysical system SGS of patient PAT is collected. In embodiments, the time series $s=s(t)$ is based on a video feed of imagery acquired by sensor device S of a patch of skin, such as in the mentioned PPG setting. Thus, the sensor device S may include a PPG camera. Raw measurements $s'= s'(t)$ acquired by sensor may be pre-processed to obtain the signal $s=s(t)$ to be processed by gating system GT. The preprocessing may be done at the sensor device S, or by a preprocessor module (not shown) on-board the gating system GT, or by such a module located elsewhere. The pre-processing may include conditioning such as a/d-conversion, amplification, signal-shaping, etc, or other pre-processing in order to derive a suitably defined measurement signal $s=s(t)$. However, in other embodiments it is the raw signal that is further processed by gating system GT, so such pre-processing (other than a/d-conversion) is not requisite herein in all embodiments. In embodiments with such pre-processing, in particular in connection with PPG or other image/video-based embodiments, pixel values in given acquired frame are averaged, weighted-averaged or are otherwise combined to arrive at a single scalar value s as per instant t, s(t), thus obtaining the measurement signal $s= s(t)$. Having said that, vector type signals rather than scalar type ones are not excluded herein. Suitable conditioning operations are described by W Wang et al in "Fundamentals of camera-PPG based magnetic resonance imaging ", published in IEEE Journal of Biomedical and Health Informatics, vol 26(9), pp 4378-4389 (2022).

In general, and as will be detailed below, the processing by gating system GT may be based on measurement signal $s = s(t)$ per instant t. Alternatively or in addition, processing may be based on a time section of the signal $s$ buffered over a certain time period $\bar{t}$, and the gating system GT operates on the so buffered values $[s(t^1), \dots . s(t^n)]_{j, \in \bar{t}}$ at once for any output produced at gating system GT's output port OUT. The time section of the series $\bar{t}$ may define a window w, on which more below. In other embodiments, the whole series is received at the input port IN of the gating component GT and this is then processed, such as in retrospective gating, although this is less preferred herein as real-time processing is mainly envisaged. Thus, the proposed system GT is preferably configured for real-time processing as the signal $s(t)$ comes in at input port IN over time $t$. Such (quasi-) real time processing, or per instant $t$ processing, or via a (short) buffering over a suitably short period $\bar{t}$, is preferably envisaged herein. Such real-time processing is preferred herein for prospective gating as mainly envisaged herein. As will be detailed below, processing involves a transformed version $Ts(t)$ of the signal s, and the above mentioned per instant t or buffered/windowed processing may also apply to the transformed signal $Ts(t)$.

For example, a suitable PPG or other image/video signal s may be constructed from raw footage s' using region of interest localization and/or automatic skin patch identification as described in *Wang et al.*. In some embodiments, signal *s* construction is based on a two-step approach: in a first step, PPG candidate signals are created per patch. Suitability of each candidate is determined, and then, in as second step, a weighted averaging over candidates is done (based on the suitability) to construct the final PPG signal s. Suitability may be quantified by some metric or objective function, depending on the task at hand. Other manners of combination instead of weighted averaging may be used instead. The two-step approach is not confined to PPG signals, but can be used instead for other image or non-image data, spatial or non-spatial, as collected by sensor S.

The pre-processing into signal *s* may also entail an encoding of the raw signal s', and this may affect the "semantics" of the measurements that form the signal *s*. For example, in PPG, the measurements may vary with blood volume at the measured site SGS. Different types of encodings may be envisaged. For example, in some encodings, the higher the signal value $s(t)$ at a given instant *t*, the higher the blood volume, whilst in some other (inverse) encodings, the higher the signal value, the lower the blood volume, and similar for other types of measurements.

A transformer TF operates on the time domain signal s, and transforms same into a time domain representation of the phase of the signal. The so transformed time series $Ts(t)$ of the phase of the signal $s$ is then processed by a bio-marker extractor BX to extract therefrom bio-marker *b* that represents, or relates to, a desired state of target TS. Such extraction may in particular include monitoring for the instant t* when the over-time changing signal s satisfies a per-defined extraction condition. For example, in embodiments, the transformed signal $T(s)(t)$ is thresholded in a thresholding operation against a threshold, such as zero to implement a zero-crossing extraction policy. However, other such extraction policies may be equally considered, largely depending on the numerical range of the transformed signal $T(s)$, its coding etc. Extraction operation may include averaging of values over a window w (on which more below) and it is the so averaged or otherwise combined values T(s) that are then thresholded.

A useful transformation operation implemented by the transformer TF has been found to be based on the Hilbert transform $\mathcal{H}(s)(t)$, as mainly envisaged herein in embodiments, although other transformations are also envisaged herein that are capable of transforming the original time domain signal measurements s(t) into another time domain signal T(s)(t) that represents, or is related to, a global phase of the signal s. The global phase may be thought to describe how various cyclical components such as frequency components that make up the components signal relate to each other. The transformer TR will be described in more detail below.

Optionally, there are filter components, such as an up-stream filter FLU and/or a down-stream filter FLD (each relative to the transformer TR), in order to smoothen the respective signal for better performance for example. For example, a low-pass filtering may be used to rid signal (s or T(s) ) off, or at least suppress, high frequency noise components as may be found in some instances in the measurements or that may be introduced as artifacts by transformer TR itself. In embodiments, there is such filtering either upstream (see up-stream filter component FLU), or down-stream (see downstream filter component FLD), but not both. However, in other embodiments, both up-stream UFL and down-stream filtering DFL is used. If both filter instances are used, at each a low-pass filter may be used, but other filter combinations are not excluded herein. Generally, the filter component(s) UFL, DFL as envisaged herein are mainly configured for noise filtering.

An optional windower W may be used to define the above mentioned processing window *w* in the transformed signal train T(s)(t). The bio-marker extractor BX operates on transformed values $T(s)(t)_{t \in w}$ in this window, as will be explained in more detail below. The extractor BX may operate per instant, or per multiple instants in the window w. The said extraction operation may be based on averaging, weighted averaging or any other ways of combining information found in the respective window w. The window may be moved over time. As mentioned above, extraction policies may be formulated in terms of thresholding the so processed (averaged, etc) phase values T(s) that are found within the timing window w. The window relates to a- given instant $t$, and extends into the past from the given instant $t$. Thus, a hold-buffering circuit may be needed. Real-time processing is ensured as the proposed windowed-setup is causal. The length of the window need not extend over the whole time series $s$.

The bio-marker $b$ *instance* as extracted herein may then be output at output port OUT and may be used for various tasks including for display on the display device DD, for storage in memory MEM or a database DB, or, as mostly envisaged herein, may be used in prospective gating. The outputting of the biomarker $b$ instance may in particular include outputting the trigger instant/timing, that is the time the biomarker was extracted/found or simply a signal that indicates that the extraction has occurred. Thus, it is the trigger instant t* of when the biomarker $b$ is extracted, or an indictor signal thereof, that may be used in a control interface CL to cause triggering a projection/detector data $\lambda$ acquisition. Thus, for some or each occurrence of bio-marker $b$, an imaging/acquisition operation of imager IA may be triggered. Thus, a sequence of bio-marker instants t* may be provided by the gating system GT to cause triggering, by suitably interfacing via control interface CL with control circuitry of imager IA, a respective data acquisition operation. For example, by issuing a suitably synchronized trigger signal based on the occurrence of biomarker $b$, an MRI pulse sequence may be issued. For example, an LG pulse sequence may be triggered based on the established instance of the bio-marker $b$.

In some embodiments of cardiac imaging, the state of the target system TS which the biomarker indicates is that of the R-peak (beginning of systolic state). Other extraction policies may allow extraction of different biomarkers that represent different states of the target system TS, such as beginning of diastolic state, etc, as required by the clinical task at hand.

As mentioned earlier, the gating system GT may not necessarily rely on the exact timing of when the bio-marker $b$ is extracted (although such embodiments are not excluded). Instead, the grating system GT may apply at output OUT, or elsewhere, a time offset $\Delta$ added to the time t* that the bio-marker $b$ was extracted, in order to account for trailing of the bio-marker in respect to the state of interest of the target system. For example, a suitable offset may be added to the timing of the bio-marker instant t* . However, in some embodiments in connection with cardiac imaging (eg for MRI or other, such as CT), the biomarker $b$ as found herein consistently to occurs after the (next) R-peak state/beginning of systolic state. However, as delay was found herein to be sufficiently low, the biomarker based on the time domain phase signal, may be used with benefit wherein in particular for gating in cardiac imaging ( MRI, CT or other).

Turning now in more detail to the bio-marker b of interest herein, this has been found to be usefully taken to be the zero (or other threshold) crossing(s) of the instantaneous phase T(s), or other global phase representation in time domain. The said phase representation in time domain may be gotten, based on, for example, by applying the Hilbert transform $\mathcal{H}$ to the original measurement signal $s$, as indeed envisaged herein in embodiments. It has been found that in the context of PPG, and given a corresponding coding of the signal s, the zero-crossings of the time domain phase represent the R-peak state of the myocardium, which of interest in cardiac imaging.

Operation of the signal transformer TF will now be explained in more detail. From the time-domain PPG or other measurement signal $s$, an analytic signal may be computed in the complex plane over time as follows:

$$\mathcal{A}(s) = s + i \cdot \mathcal{H}(s) \qquad (1)$$

wherein $i^2$ = -1, and operator $\mathcal{H}$ denotes the Hilbert transform.

The time domain phase signal (such as the instantaneous phase signal) of the PPG or other measurement signal $s$ may be computed following (1) by taking the phase of the analytical signal:

$$T(\text{s}) = \varphi(s) = \arg \mathcal{A}(s) = \arctan \left(\frac{\mathcal{H}(s)}{s}\right) \qquad (2)$$

The PPG signals can be described in a harmonic series with dominant amplitude at the fundamental frequency. The phase trajectories are expected to resemble a monotonically increasing function, being nearly linear in nature, with average derivative corresponding to the fundamental frequency and wrapping around $[-\pi, \pi]$. Hence any arbitrary given value of $\varphi$ corresponds to a state of the heart contraction. Hence one can derive an MRI trigger t* = $t_k$ by solving $\varphi(s)(t_k)$ = $k$. It was found that the value $k$ = 0 was a suitable threshold value to detect since it may correspond to a maximum amplitude in the time-domain signal. This has been verified in practice. It has been found that delay between the ECG R-peak and the zero-crossing of the instantaneous phase is stable for a given patient, which make this approach a suitable for cardiac MRI triggering for example.

The transformer TR may operate per time instant t dynamically, so the transformed signal is computed *Ts(t)* per time $t$ (at each or some current time $t_0$).

Thus, the transformed signal *T(s)* as produced by transformer TF, eg as per eqs (1),(2) above, is in some embodiments the argument in the complex plane of the analytical signal representation of signal s, based on the Hilbert transform of the signal s, or on any other such transform.

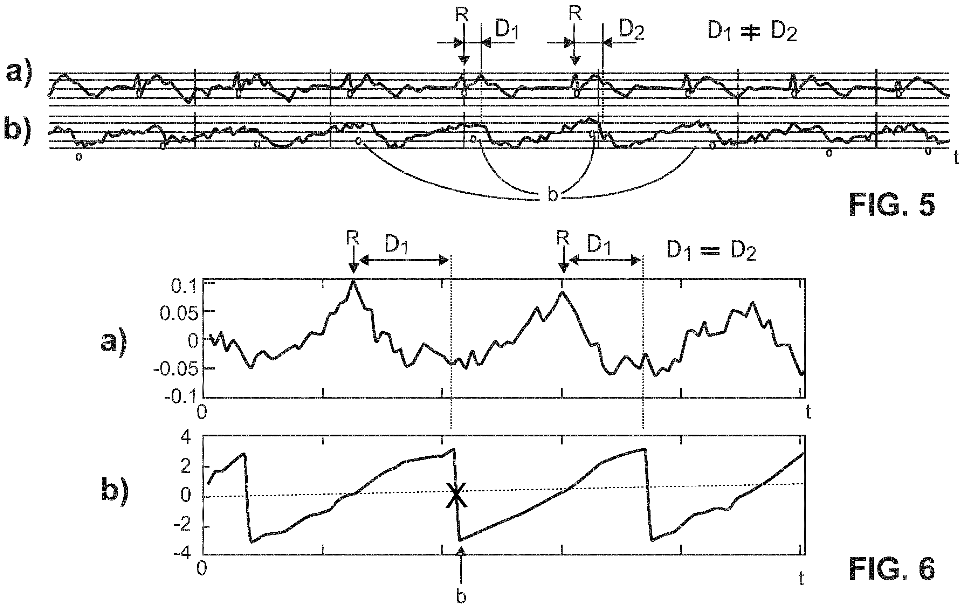

The time domain phase representation T(s) may be conceptualized as a phasor entity, which traces out the phase angle in the complex plane, relative to a reference position, such as the real or complex axis (3 or 12 o'clock position). The analytical signal or phasor encapsulates all the information in the original time domain signal into a complex number. It is thought that by so encapsulating, or mapping out, the original time domain signal s into a two-dimensional space (such as the complex plane), the information is "spread" out across such dimensions, and thus the global phase aspect of the time-domain measurement signal can be laid bare with sufficient explicitness, as can be seen in the time curves as shown in Figs. 5, 6 to which reference is now made. Such spreading out into a space of at least two dimensions may also be envisaged by using other transforms than the Hilbert transform based analytical signal.

Fig. 5a) shows an ECG trace with R-peaks indicated. The ECG trace presents the true states of the target system, with R-peak ("R" in the Figure) representing onset of systolic state, and hence low blood volume at surrogate site SGS (eg, patch of skin at forehead, temples, etc). Time curve b) shows the time domain measurements s, that is, without operation of transformer TR. The instant of low blood volume (blood depletion, shown as small circles in curve b) ) has been found to be a useful bio-marker which is related to the *R*-peak in the target system. However, because of jitter, there is usually a variable time offset $\Delta$ between the two (the R-peak and the instant of low blood volume at surrogate SGS). Thus, the offset may not be equal across instances, which makes a reliable gating difficult in the signal representations of Figs. 5a), b).

The situation is improved, thanks to transformer TR, as demonstrated by the time curves in Figs. 6a), b). Curve a) again shows a trace of the ECG with the R peaks, whilst curve b) now shows the transformed-into instantaneous phase (time domain global phase), based on the analytical signal which may conceptualized by the Hilbert transform. Thus, Fig. 6b) may be understood as the transform of Fig. 5A's PPG signal *s* (or other measurement signal, not necessarily PPG). Now, the zero crossings of the instantaneous phase (shown as "*X*" in Fig. 6b)) in the surrogate system SGS, and the R-peaks of the target system TS are at a defined, constant offset $\Delta$ over time. This allows for consistent, reliable gating, and hence better imaging, with less wear and tear on imaging equipment, better patient comfort and higher imaging throughput.

It will be understood herein than the ECG trace above at Figs. 5A, 6A was merely used for illustration of ground truth, and that the proposed gating system GT, whether based on PPG or on surrogate measurements s, can be used instead of the cumbersome and interfering ECG method. ECG equipment and methodology greatly hinders imaging workflows, especially in tight, closed-bore systems, such in some MRI (see Fig. 2) or CT imager IA designs. Instead, the proposed system GT, thanks to its transformer TR, can replace such ECG-based gating systems and is operable for blind gating (that is, without such ECG).

In the PPG vs R-peak case, it is seen in Figs. 5,6 that the zero-crossings in the phase time domain representation consistently occur after the R-peak. The same consistent situation may also hold true of for other type of measurements, or other features of the QRS complex, other than R-peak. As mentioned earlier, the zero-crossing is merely an example, and the thresholding depends on the manner in which the signal s is encoded (see the above preprocessing). For example, it may be that a high value in the phase domain represents a low blood volume, or the other way around. Any specific manner of signal encoding/construction of the s signal may be used herein, so long as this is used consistently. Thus, in some such embodiments, instead of a zero-crossing, there may be a thresholding against a value other than zero, as needed, to so find the biomarker b in time domain phase T(s) for gating purposes. It may be noted that even if a non-zero threshold is used, by shifting this situation may be reformulated as a zero-crossing. Thus, the illustration of the principles disclosed herein using the zero-crossing extraction policy does not limit the generality of the present disclosure.

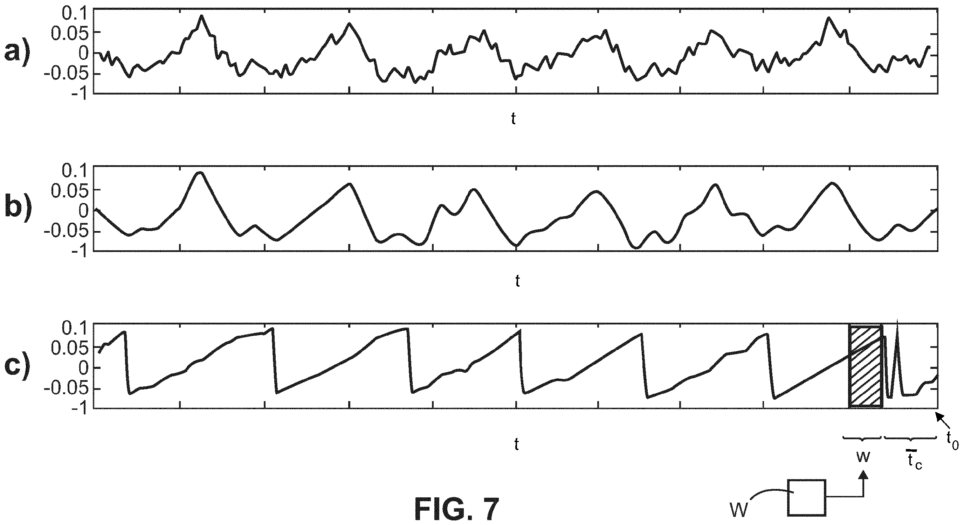

Fig. 7a)-c) illustrate the windowing operation W, as mentioned earlier. As can be seen, Figure a) shows again an ECG trace, whilst curve in Figure b) shows a low-pass filtered version of curve a). Fig. 7c) shows the time domain phase representation as derived from the analytical signal in connection with the Hilbert transform, or from any other suitable transform TR. As can be seen, close to the current time t0, the signal deteriorates which has to do with the manner in which the time domain phase is computed in some embodiments and/or with the manner the filtering FLD operation works, if applied. Thus, it is proposed herein to configure the windowing w, so that its terminal point expires before the current time *to,* and outside a clearance or safety margin $\bar{t}_c$ between current time and expiry of window w. Any one, more than one, or all of i) the transformation TR, ii) zero crossing or, more generally, feature extraction, iii) filtering FLD may be done in the windowing interval. The operations such as extraction, filtering, transformation etc, or other processing, may then use one or more phase values within the window w, as required.

The window width defines a strength of the smoothing on the instantaneous phase. A shorter window results in higher temporal resolution while a longer window is advantageous when the phase computation TR is noisy. It was found that a window of about 130-170 ms wide gave a good trade-off. The midpoint or other localizer of the window may be chosen as a trade-off between robustness and maximal delay that the system can support. Preferably, a localizer for use in an MRI setting is adapted to the acquisition sequence. The longer the delay, the further away the localizer should be from the so-called "edge-effects" at t0 of real-time processing as illustrated in the Fig. 7. The windowing may be dynamically adapted over time. The windowing may allow for improved real-time processing, in particular in prospective gating as mainly envisaged herein in embodiments.

Specifically, for the transformer TR, phase recovery and/or filtering outside the safety margin $\overline{t_c}$ is stable enough, and it may be beneficial to average within this window w for more robustness. Hence in the proposed setup, system with windowing, there are two more system parameters there is the location/center point of this analysis window w, (a system delay with which the trigger is detected), and the window width, to smooth out outliers.

The numerical computation of the Hilbert transform, if used by transformer TR, may be based on the Fourier transform of the original signal s into frequency domain, and performing there a +/-90° frequency shift for negative and positive spectral component frequencies, respectively, and by inverse Fourier transformation back into time domain. A fast Fourier transform algorithm (FFT) may be used for computational efficiency. In other embodiments, the Hartley transform may be used to compute the Hilbert transform. See for example, R NBracewell in "The Fourier Transformation and Its Applications", Chapter 13, 3rd edition, McGraw Hill, (2000).

In some embodiments, an outcome of the transformation TR is that the positive frequencies are doubled, and that the negative frequencies are removed from time domain measurement signal s.

It may be remarked in this connection, that the representation of the time domain phase signal in terms of the Hilbert transform may be understood as conceptual. Thus, the Hilbert transform as such may not need to be compute explicitly, such as in the Fourier transform based embodiments. Thus, with Fourier or other methods, the time domain phase representation (such as the analytical signal), may be obtained without using the Hilbert transform as an intermediate form. Having said that, computational approaches are also envisaged that rely for their implementation on eq (1), (2) and thus may indeed call for such an explicit computation of the Hilbert transform $\mathcal{H}$ .

Reference is now made to Fig. 8 which illustrates features that may be used for delay $\Delta$ prediction in respect of the PPG (or other) waveform s. As indicated above, the delay is one between occurrence of the biomarker b extracted, and the state of the target TS which is of interest and associable with the biomarker b.

As mentioned, in some MRI acquisition pulse sequences, a preparation pulse (and hence additional time) is required. For example, in end-diastole state for LGE sequences such preparation pulse may be needed., similar for some systolic centered imaging protocols. Thus, predicting the (expected) delay $\Delta$ (time difference) between ECG R-peak and the zero-crossing PPG trigger is of consideration herein.

In Fig. 8, the dotted-dashed line represents the R-peak instant, whilst the dotted line is the zero-crossing instant of the time domain phase (eg, instantaneous phase). In order to fully replace the ECG modality, the estimation of delay $\Delta$ should preferably only rely on data ("features") from the PPG signal. It was found that using more than one PPG feature(such as two, or three), it may be possible to derive low-rank models to estimate this delay for a given patient PAT. Some such features are illustrated in the said Fig. 8, to which reference is now made in more detail. Again, the delay $\Delta$ is illustrated for a PPG signal *s,* but as said before, this is not limiting, and could be applied also to other blood volume measurement s, or other measurements, not necessarily related to blood volume.

Not only can the proposed marker b derived accurately from the time domain phase signal *Ts = T(s)* , but it is also the relation between the phase and target state of interest (such as the R-peak) that can be predicted using PPG data only. For the case of using the positive-going zero-crossing *Z* as biomarker *b,* the delay $\Delta$ can be accurately predicted by using suitable features in the time domain measurement signal *s* (eg, PPG-signal), and its associated time domain phase signal *T(s)*. A non-exhaustive illustration of these features are illustrated in Fig. 8, with depth *d* defined as the ratio between the range of the PPG waveform and its mean level.

The features F and D denote respectively the up-going and down-going zero crossing of the PPG signal. M is a maximum of the PPG waveform, and Z denotes the up-going zero crossing of the associated phase signal as preferably used herein as biomarker b, but, as said before, other biomarkers may be used instead.

With one or two of these features only, accurate predictions of $\Delta$ may be obtained. In one embodiment, a time difference between the phase marker Z and the maximum M of the PPG signal s may be used as an estimate. In embodiments, a second PPG feature is used. For example, this second PPG feature can be taken i) as the time difference between the down- and up-going zero-crossing in the PPG signal (F and D), or i) as the time difference between the PPG maximum and the up-going zero-crossing in the PPG signal (M and Z), or iii) the time difference between phase marker and up-going zero-crossing in the PPG signal.

In embodiments, a linear combination of any two of these parameter pairs can be used to predict $\Delta$. Other feature combinations, or combinations that use three or more features are also envisaged. In general, for better responsiveness, a low number of features is used, such as one, two three, etc. Preferably, less than 5 are used.

A slightly less accurate prediction can be obtained by determining a suitable offset and the (optionally, scaled) time difference between the phase marker Z and the maximum of the PPG signal M. The above-mentioned features, whether single or pair, can be extracted in a preparatory/calibration phase from a PPG curve, collected from patient PAT prior imaging, in order to establish a series of estimated delays $\Delta_j$, and these may then be averaged or otherwise combined to estimate the delay $\Delta$.

The delay $\Delta$ may be pre-computed, and may then be used for given patient or other patients in imaging for gating. In some embodiments, the delay may be used to refine time domain phase based estimate t* such as the zero-crossing. However, in some embodiments (such as when the t* occurs after the state of interest, such as in relation to the R-peak), the delay may be used for confirmation of the delay. For example, cardiac MRI the delay may be confirmed to be low enough for a given patient, such that the intended MRI pulse sequence fully fits in the IBI for the given patient (see Fig. 10 below for more details on this). A confirmatory signal may be issued if this is the cause by operating a transducer, such as a display device, a lamp, a speaker, etc. Instead of or in addition, the confirmatory signal is used as an additional gating criterion. Thus, data acquisition is triggered when biomarker *b* is extracted/found and the delay is found to be sufficiently low. Thus, the delay computation is not a one off, but is done dynamically repeatedly at some or all instants, when biomarker is extracted. Alternatively, an alert signal is issued. Knowing the delay $\Delta$ may also allow aligning the acquired data in the triggering with the R-peak or state of electrification, for yet improved ECG blind synchronization. The computation of the delay is optional, but is preferred in some embodiments.

The delay may be estimated for each patient individually in the said calibration phase, which can be done quickly and will not disturb clinical workflow.

As can be seen in Fig. 8 (and as will be illustrated latter below in Fig. 10), using the time domain phase-based gating approach, the delay $\Delta$ between, eg, zero-crossing of the time domain phase, and the R-peak instant is low. The time domain phase-based biomarker b (such as the said zero-crossing) may occur before or after the R-peak. But it has been found to be sufficiently close to the R-peak, so that it may be used with benefit herein. For example, delay $\Delta$ is so low in the proposed setup, that in many MRI pulse sequences (even those that need a preparation pulse), fit in its entirety into an IBI.

Thus, the gating system GT may include a delay estimator module DEM to specifically estimate the delay $\Delta$ between biomarker b instant, and instant of state of interest in target TS), using any of the pair of features as explained above. Alternatively, the delay is estimated based on historical ECG data from the patient or from other patients. As mentioned earlier, machine learning (ML) may be used to predict delay based on patient characteristics for example. Such delay $\Delta$ may be established from clinical experience, or may be assumed herein to be known, by whichever way. Alternatively, and preferably, a low-order model, such as a linear model, averaging, etc, may be used to predict delay as mentioned above.

Estimation of delay $\Delta$, tailored to the proposed time-domain phase driven gating is useful, because this allows retrofitting for present gating purposes, existing (instructions for) data acquisition operations, such as MR pulse sequences. For example, in sequences like LGE (Late Gadolinium Enhancement) or others, the aim is to obtain images in a specific cardiac state. For that, presently, there exist tables that may prescribe specific trigger delay amounts (eg, in *ms)*, which the system has to wait after an observed feature, such as the mentioned R-peak. However, the delay $\Delta$ achievable with the proposed time domain phase, based on PPG or other, rather than on the originally intended ECG, will be different from the originally intended R-peak triggering. This may be because them eg, R-peak is, as such, no longer observed directly, as the proposed PPG or other measurement signal s is to replace the ECG setup. Knowing the delay $\Delta$ as estimated herein for the specific R-peak-to-biomarker b, thus allows reusing a good number of the existing stock of old MRI sequences "as is", by merely adapting (preferably automatically) the originally prescribed trigger delays. This allows even better integration of the proposed method into existing clinical workflows or imaging setups.

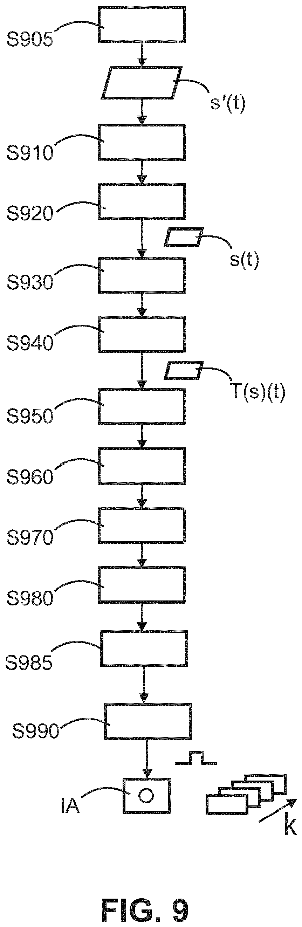

Reference is now made to the flow chart of Fig. 9 which illustrates steps of the above-described gating operation, based on time domain phase representation of the measurement signal s, acquired in relation to the surrogate system. The method may be used in gating operations, such as prospective or retrospective, in relation to imaging by an image apparatus of target system. State changes at or of the target system TS are assumed to be functionally related to counterpart states changes at or of the surrogate system. The state changes at or of the surrogate system are measured by the measurement signal s. The method is based on using a suitable transform that transforms the initial measurement time domain signal s into the said s time domain phase representation. The so obtained time-domain phase representation T(s) may then be used in lieu, or in addition to, the original signal *s.* The time domain phase signal has been found to be useful for reliably extracting based thereon a suitable bio-marker b as described above, which can then be used for gating, for example in controlling an image data acquisition operation in respect of the associated target system TS.

At step S905, raw data time domain measurements s'=s'(t) at or of the surrogate system is collected. The raw data is collected preferably remotely and non-invasively, such as image data, by using visible or invisible light, ultrasound (US), IR, NIR, but also non-radiation based modalities are envisaged, such as scale or piezoelectric measurements in ballistic (recoil-based) applications, etc.

At optional step S910, the raw data may be pre-processed to obtain a pre-processed time domain signal *s=s(t)* of measurements, to be further processed herein, instead of the raw data. The pre-processing may include conditioning or averaging or otherwise combining the (eg, spatial) raw data per instant into signal s at instant t. The preprocessing may also include encoding. It is the so pre-processed measurements *s* collected in relation to the surrogate system which are then received at step S920 for gating purposes.

At step S930 there is an optional filtering step such as low-pass filtering to remove noise components from received signal s(t).

At step S940 the, optionally filtered, measurement signal s is transformed to obtain a time domain phase representation *T(s(t)) = Ts(t),* such as by computing the instantaneous phase based on the Hilbert transformation of the time domain signal s(t). An analytical signal representation may be used. Fourier methods may be used in step S940. It may not be necessary herein in all embodiments to explicitly compute the Hilbert transform.

At step S950 there is an optional, first or second (if the (first) filtering S930 step is used), filter step that filters the time domain phase representation signal T(s). Again, or now, a low-pass filtering may be used. Both filtering steps, the up-stream and down-stream steps S930, S950, respectively, are optional, or either one may be used, or both may be used, as required.

The time domain phase representation signal T(s), whether filtered or not, is monitored and at step S970, the bio-marker is extracted in an extraction event from the time domain phase signal T(s)(t) as per step S940. Such extraction event occurs, if the monitored signal T(s)(t) fulfills one or more conditions as per an extraction policy. Thresholding, such as zero-crossing, may be one such policy. If the signal does so fulfill the said policy, the said extraction event can be said to have occurred.

Optionally, the extraction operation may be based on windowing the time domain phase representation signal at step S960. Thus, the extraction S970 may be based on plural or a single value of T(s)(t) as buffered in window w.

At step S980 the time instant t* at which bio-marker b is so extracted is output and made available for further processing, as required. The time instant t* is thus of such an extraction event. Instead of, or in addition of the timing, another indicator signal is output, such as an agreed number ("0" or " 1", or any other number), or any other data item, such as a pulse, that is agreed to be indicative of the extraction event.

In particular, at step S990, a gating operation may be done based on timing t* or other extraction event indicator signal as issued at step S980. Optionally, the method may include a delay estimator step S985, or said step may be part of output step S980. The delay estimator produces an estimate $\Delta$ of a delay between occurrence instant of extracted biomarker, and the intended state of the target TS one wishes to gate for. The processing may be based on occurrence instants one or more features of the measurement signal s and/or of the transformed into time domain phase representation Ts. A linear combination based on merely two or more than two such features may be used to estimate delay. The delay $\Delta$ may be computed at any time, and not necessarily between steps S990 and S980. The delay may be used/accounted for in the gating operation. The delay me be pre-computed, such as in pre-preparatory phase. It may be computed once and used of multiple (eg, similar patients), or it may be computed for a given patient individually. When the delay is computed, this can be displayed. The delay may be compared with a data acquisition latency of imaging apparatus, and an alert signal is issued, if the delay happens to be too long, or no data acquisition operation is issued for extracted biomarker at that instant. Thus, the delay computation S985 may be used a watchdog for each or some instants, whenever a biomarker is extracted at S970. Only when the delay is found sufficiently low, a data acquisition is triggered at step S990. In addition, or instead, the estimated delay is used to adapt existing data acquisition instruction that had their own pre-specified trigger delays, not initially envisaged for use in the proposed time-domain phase signal based gating. For example, the estimated S985 delay may be used to adapt such existing trigger delays, eg by adding, subtraction, or by replacement of the existing trigger delay by the estimated delay $\Delta$. Thus, an existing stock of MRI pulse sequences, originally intended to be driven by ECG signals, may be reused in the proposed time-domain phase representation Ts(t) driven gating setup. The method may thus further include a step of adapting such existing data acquisition instructions, and use those in the following gating operation S990.

The gating operation S990 may include controlling (eg, triggering) a data acquisition operation of imaging apparatus in respect of patient, such as in prospective gating. In such gate data acquisition operation projection data $\lambda$ may be acquired (in CT. MRI, nuclear imaging, US, etc) from which imagery for the target state of the target TS may be obtained. In addition, or instead, in retrospective gating embodiments, the processing there may include tagging with tags "$\kappa$", based on extraction times t* (optionally with delay $\Delta$ accounted), a stream of data $\lambda$ acquired by the imaging apparatus. The tags may be incorporated as metadata, such as into header files of individual frames. Other processing steps may include storing, displaying or otherwise processing the tagged frames, and/or the extracted time t* for biomarker b, as needed. As mentioned, the acquisition operation (such an MRI pulse sequence) may have its original trigger delay adapted by the estimated S985 delay, and it is the so adapted data acquisition operation that is used in gating step S990.

In an additional further step (not shown), the method may include reconstructing, from the acquired or tagged image data $\lambda$, the imagery in image domain. The imagery so reconstructed may represent the target system in the desired target state, associated with the extracted biomarker.

It has been found herein that the phase characteristic of the fundamental as derived from the PPG measurement of the forehead or elsewhere, is a kind of mean characteristic, ignoring details of the waveform s. However, it is known that the systolic and diastolic part of the cardiac cycle responds differently to a change in IBI. This is not the case when considering the phase response of the fundamental only. It will result in a straight line for the phase characteristic. Thus, it is preferred herein to not merely use the fundamental spectral frequency component as per the original signal s, but to leave other frequencies in the signal for processing by gating system, as this allows for an exact timing for relating some PPG marker *b* (from the fundamental) to the R-peak position. Removing, by filtering, all such frequency domain components other than the fundamental is thus not recommended herein, as this would jeopardize such timing.

Fig. 10 is an illustration of a beneficial effect the above-described gating system GT and method in the context of MRI. The bottom part shows a timing diagram of a data acquisition operation AO in MRI. The data acquisition operation AO may comprise instructions including a pre-pulse/preparatory pulse PP and the acquisition pulse AP, with a modality specific latency or preparation delay L in between the two. It may well happen, were it not for the proposed system GT and method, that the duration of the acquisition operation AO does not fit entirely into the length of the IBI which is undesirable, as this may preclude gating for a state of interest , such as the systolic state SS or the diastolic state DS of heart as the target TS, and may thus compromise IQ. But thanks to basing the triggering of the acquisition operation AO on the time domain phase signal (eg, its zero crossing "*X*" or other thresholding), the delay $\Delta$ is low enough so that the duration of the acquisition operation AO in its entirety fits into the length of the IBI. Thus, the triggering point *X* /biomarker *b* is not only reliable, but also close enough to the systolic state SS, so that there is sufficient time for the acquisition operation AO to conclude within the IBI.

Thanks to the proposed time-domain phase gating, the delay $\Delta$ between R-peak and trigger point t* is so low, that a good number or MRI pulse protocols can be made to fit into IBI. The proposed method is hence applicable in particular to all those MRI pulse protocols that can be so accommodated, thanks to the low delay $\Delta$.

The components of the gating system GT may be implemented as one or more software modules, run on one or more general-purpose processing units PU such as a workstation associated with the imager IA, or on a server computer associated with a group of imagers.

Alternatively, some or all components of the gating system GT may be arranged in hardware such as a suitably programmed microcontroller or microprocessor, such an FPGA (field-programmable-gate-array) or as a hardwired IC chip, an application specific integrated circuitry (ASIC), integrated into the imaging system IA. In a further embodiment still, the gating system GT may be implemented in both, partly in software and partly in hardware.

The different components of the gating system GT may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope. Such reference signs may be comprised of numbers, of letters or any of alphanumeric combination.

## Claims

1. A gating system (GT) for data acquisition by an imaging apparatus (IA) in respect of a target biophysical system (TS), the system comprising:

   an input interface (IF) through which is receivable a time-domain phase representation of a time domain signal of measurements of a surrogate biophysical system (SGS), obtainable by a sensor (S) device capable of non-ionizing operation;
   a biomarker extractor (BX) capable in at least one extraction event to extract, from the time-domain phase representation, at least one instance of a biomarker that is associable to a state of the target biophysical system; and
   an output interface (OUT) for providing, for a data gating operation, an indication for the at least one extraction event.

2. System of claim 1, wherein the said data gating operation is prospective or retrospective, wherein the prospective gating operation include triggering a data acquisition operation by the imaging apparatus (IA), based on the provided indication of the at least one extraction event, wherein the retrospective gating operation includes tagging data acquired by the imaging apparatus (IA), based on the provided indication of the at least one extraction event.

3. System of claim 1 or 2, wherein the sensor device (S) is arrangeable for contactless and/or remote operation.

4. System of any one of the previous claims, wherein the time-domain phase representation of the phase is computable by a transformer (TF) capable of effecting a Hilbert transform of the time domain signal of measurements.

5. System of any one of the previous claims, wherein the system comprises a filter (FD, FLU) capable of low-pass filtering the time domain signal of measurements, and/or of low-pass filtering the receivable time-domain phase representation.

6. System of any one of the previous claims, wherein the extractor (BX) is capable of such extraction operation based on a thresholding, and/or wherein the extractor (BX) is capable of such extraction operation based on processing data of the time domain phase representation over a time-domain window.

7. System of claim 6, wherein an endpoint of such window is situated in time domain at a margin away ($\overline{t_c}$) from a current time ($t_0$) of the time domain phase representation.

8. System of any one of the previous claims, including a control interface (CL) capable to so cause triggering of an acquisition operation by the imaging apparatus (IA), based on the provided indication of the at least one extraction event.

9. System of any of any one of the previous claims, wherein the imaging apparatus (IA) is of the tomographic type, in particular of the magnetic resonance type, or of the projection-domain type.

10. System of any one of the previous claims, wherein A) the target (TS) or surrogate (SGS) biophysical system includes any one or more of: i) the cardiac system, ii) the respiratory system, iii) and the gastrointestinal system, and/or B) wherein the surrogate biophysical system (SGS) includes a patch of skin of an imageable patient (PAT).

11. System of any one of the previous claims, wherein the sensor device (S) is configured for sensing radiation, such as any one of visible light, IR, NIR, laser (eg, LIDAR), ultrasound, or of sensing any one of an acoustical signal, mechanical pressure or force.

12. Imaging arrangement (MIA), including the system of any one of the previous claims, and further including one or more of i) the imaging apparatus (IA), and ii) the sensor device (S).

13. A gating method for data acquisition by an imaging apparatus (IA) in respect of a target biophysical system (TS), the method comprising:

receiving (S920) a time-domain phase representation of a time domain signal of measurements of a surrogate biophysical system (SGS), obtainable by a sensor (S) device capable of non-ionizing operation;
in at least one extraction event, extracting (S970), from the time-domain phase representation, at least one instance of a biomarker that is associable to a state of the target biophysical system; and
providing (S980), for a data gating operation, an indication for the at least one extraction event.

14. A computer program element, which, when being executed by at least one processing unit, is adapted to cause the processing unit to perform the method as per claim 13.

15. At least one computer readable medium having stored thereon the program element of claim 14.

**FIG. 1**

FIG. 2

**FIG. 3**

**FIG. 4**

FIG. 5

$D_1 \neq D_2$

a)

b)

FIG. 6

$D_1 = D_2$

a)

b)

**FIG. 7**

EP 4 454 549 A1

**FIG. 8**

**FIG. 9**

FIG. 10

Europäisches
Patentamt

European
Patent Office

Office européen
des brevets

# EUROPEAN SEARCH REPORT

Application Number

EP 23 16 9779

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WANG WENJIN ET AL: "Fundamentals of Camera-PPG Based Magnetic Resonance Imaging", IEEE JOURNAL OF BIOMEDICAL AND HEALTH INFORMATICS, IEEE, PISCATAWAY, NJ, USA, vol. 26, no. 9, 17 December 2021 (2021-12-17), pages 4378-4389, XP011919507, ISSN: 2168-2194, DOI: 10.1109/JBHI.2021.3136603 [retrieved on 2021-12-20] * page 4378 - page 4385; figure 7 * ----- | 1-15 | INV. A61B5/024 A61B5/00 A61B5/055 |
| X | EP 4 123 575 A1 (KONINKLIJKE PHILIPS NV [NL]) 25 January 2023 (2023-01-25) * paragraphs [0012] - [0018]; figures 1, 6 * ----- | 1-15 | |
| X | US 2013/211235 A1 (STOUGHTON ROBERT [US] ET AL) 15 August 2013 (2013-08-15) * paragraphs [0002] - [0010]; figure 1 * ----- | 1-15 | |

TECHNICAL FIELDS
SEARCHED (IPC)

A61B
G06T

The present search report has been drawn up for all claims

| Place of search | Date of completion of the search | Examiner |
|---|---|---|
| The Hague | 14 August 2023 | Faymann, Juan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...............................................................................
& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 23 16 9779

This annex lists the patent family members relating to the patent documents cited in the above-mentioned European search report.
The members are as contained in the European Patent Office EDP file on
The European Patent Office is in no way liable for these particulars which are merely given for the purpose of information.

14-08-2023

| Patent document cited in search report | | Publication date | Patent family member(s) | | Publication date |
|---|---|---|---|---|---|
| EP 4123575 | A1 | 25-01-2023 | EP | 4123575 A1 | 25-01-2023 |
| | | | WO | 2023001997 A1 | 26-01-2023 |
| US 2013211235 | A1 | 15-08-2013 | US | 2011074409 A1 | 31-03-2011 |
| | | | US | 2013211235 A1 | 15-08-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LAURENT GIOVANGRANDI et al.** Ballistocardiography - A Method Worth Revisiting. *Conf. Proc. IEEE, Eng MedBiol. Soc,* 2011, 4279-4282

- **W WANG et al.** Fundamentals of camera-PPG based magnetic resonance imaging. *IEEE Journal of Biomedical and Health Informatics,* 2022, vol. 26 (9), 4378-4389
- **R NBRACEWELL.** The Fourier Transformation and Its Applications. McGraw Hill, 2000